# EUROPEAN PATENT APPLICATION

(11) **EP 1 637 151 A1**
(43) Date of publication of application: **22.03.2006**
(21) Application number: 04726828.9
(22) Date of filing: 09.04.2004
(51) Int. Cl.: A61K 36/00, A61K 31/7008, A61K 31/726, A61P 19/02, A23L 1/30, A23K 1/16

(54) **PREVENTIVE OR REMEDY FOR ARTHRITIS**

(30) Priority: 11.04.2003 JP 2003107405
(71) Applicant: KYOWA HAKKO KOGYO CO., LTD., Chiyoda-ku, Tokyo 100-8185 (JP)
(72) Inventor: KAMIYA, Toshikazu, c/o Healthcare Research Lab., Tsukuba-shi, Ibaraki 305-0841 (JP); NAKAGIRI, Ryusuke, Chapel Hill, NC 27516 (US)
(74) Representative: Vossius & Partner
(86) International application number: PCT/JP2004/005115
(87) International publication number: WO 2004/091642

(57) **Abstract**

An object of the present invention is to provide a preventing agent or a treating agent for arthritis or to provide foods and drinks, additives for foods and drinks, feeds or additives for feeds for prevention or treatment of arthritis.

In order to achieve the above object, the present invention provides a preventing or treating agent for arthritis or foods and drinks, additives for foods and drinks, feeds or additives for feeds for prevention or treatment of arthritis comprising plant belonging to the genus Hydrangea or an extract of the plant and amino sugar or a salt thereof and/or glycosaminoglycan or a salt thereof as active ingredients.

## Description

### Technical Field

The present invention relates to an agent for preventing or treating arthritis and to foods and drinks, additives for foods and drinks, feeds and additives for feed for prevention or treatment of arthritis.

### Background Art

Hydrangea macrophylla Seringe var. Thunbergii Makino belonging to the genus Hydrangea is an allied species of hydrangea and said to have been made through breeding of Hydrangea macrophylla SER var. acuminata in the Edo era. Hydrangeae Dulcis Folium is prepared by fermentation of the harvested leaves and branch ends of Hydrangea macrophylla Seringe var. thunbergii Makino, followed by drying.

Hydrangeae Dulcis Folium or its extract has long been used as a corrective (sweetener) in pills or as a raw material for buccal refrigerants. The aqueous extract of Hydrangeae Dulcis Folium has been used as a sweetener in the form of liquid or powder. The sweetening component is phyllodulcin.

Hydrangeae Dulcis Folium has been known to have an anti-coccidium activity, anti-fungal activity, anti-ulcer activity, anti-allergic activity, hypercholesterolemia-suppressing activity, anti-periodontal bacteria activity, anti-oxidative activity, and the like (Abstracts of Papers presented at the 2nd Symposium on Medicines and Foods, p.85, 1999). The extract of Hydrangeae Dulcis Folium is known to have a cholagogic effect (Yakugaku Zasshi (Journal of Pharmaceutical Society of Japan), vol. 114 , no. 6, 401-413, 1994). In addition, the extract of Hydrangeae Dulcis Folium is also known to show in vitro the effect of inhibiting lipid peroxidation in liver microsomes (Natural Medicines, 1995, vol. 49, no. 1, 84-87).

Meanwhile, plant belonging to the genus Hydrangea or an extract of the plant has been known to have a preventing or treating effect for arthritis (WO 02/102,396). It has been also known that chondroitin sulfate, glucosamine, and the like, which are constituting components of cartilage have a treating effect for arthritis (Japanese Patent No. 2,971,579).

However, no composition comprising a combination of plant belonging to the genus Hydrangea or an extract of the plant and amino sugar or a salt thereof and/or glycosaminoglycan or a salt thereof has been known and it has not been known that the composition shows a synergistic preventing or treating effect for arthritis.

### Disclosure of the Invention

An object of the present invention is to provide a preventing agent or a treating agent for arthritis or to provide foods and drinks, additives for foods and drinks, feeds or additives for feeds for prevention or treatment of arthritis.

The present invention relates to the following (1) to (27).
(1) A preventing or treating agent for arthritis which comprises plant belonging to the genus Hydrangea or an extract of the plant and amino sugar or a salt thereof and/or glycosaminoglycan or a salt thereof as active ingredients.
(2) The preventing or treating agent for arthritis according to (1), wherein the plant belonging to the genus Hydrangea is Hydrangea macrophylla Seringe var. Thunbergii Makino.
(3) The preventing or treating agent for arthritis according to (2), wherein the plant of Hydrangea macrophylla Seringe var. Thunbergii Makino is Hydrangeae Dulcis Folium.
(4) The preventing or treating agent for arthritis according to any of (1) to (3), wherein the amino sugar is glucosamine or a salt thereof.
(5) The preventing or treating agent for arthritis according to any of (1) to (4), wherein the glycosaminoglycan is chondroitin sulfate or a salt thereof.
(6) A food and drink or an additive for foods and drinks comprising plant belonging to the genus Hydrangea or an extract of the plant and amino sugar or a salt thereof and/or glycosaminoglycan or a salt thereof.
(7) The food and drink or the additive for foods and drinks according to (6), wherein it is used for prevention or treatment of arthritis.
(8) The food and drink or the additive for foods and drinks according to (6) or (7), wherein the plant belonging to the genus Hydrangea is Hydrangea macrophylla Seringe var. Thunbergii Makino.
(9) The food and drink or the additive for foods and drinks according to (8), wherein the plant of Hydrangea macrophylla Seringe var. Thunbergii Makino is Hydrangeae Dulcis Folium.
(10) The food and drink or the additive for foods and drinks according to any of (6) to (9), wherein the amino sugar is glucosamine or a salt thereof.
(11) The food and drink or the additive for foods and drinks according to any of (6) to (10), wherein the glycosaminoglycan is chondroitin sulfate or a salt thereof.
(12) A Feed or an additive for feeds comprising plant belonging to the genus Hydrangea or an extract of the plant and amino sugar or a salt thereof and/or glycosaminoglycan or a salt thereof.
(13) The feed or the additive for feeds according to (12), which is used for prevention or treatment of arthritis.
(14) The feed or the additive for feeds according to (12) or (13), wherein the plant belonging to the genus Hydrangea is Hydrangea macrophylla Seringe var. Thunbergii Makino.
(15) The feed or the additive for feeds according to (14), wherein the plant of Hydrangea macrophylla Seringe var. Thunbergii Makino is Hydrangeae Dulcis Folium.
(16) The feed or the additive for feeds according to any of (12) to (15), wherein the amino sugar is glucosamine or a salt thereof.
(17) The feed or the additive for feeds according to any of (12) to (16), wherein the glycosaminoglycan is chondroitin sulfate or a salt thereof.
(18) A preventing or treating method for arthritis, which comprises administering plant belonging to the genus Hydrangea or an extract of the plant and amino sugar or a salt thereof and/or glycosaminoglycan or a salt thereof.
(19) The preventing or treating method for arthritis according to (18), wherein the plant belonging to the genus Hydrangea is Hydrangea macrophylla Seringe var. Thunbergii Makino.
(20) The preventing or treating method for arthritis according to (19), wherein the plant of Hydrangea macrophylla Seringe var. Thunbergii Makino is Hydrangeae Dulcis Folium.
(21) The preventing or treating method for arthritis according to any of (18) to (20), wherein the amino sugar is glucosamine or a salt thereof.
(22) The preventing or treating method for arthritis according to any of (18) to (21), wherein the glycosaminoglycan is chondroitin sulfate or a salt thereof.
(23) Use of plant belonging to the genus Hydrangea or an extract of the plant and amino sugar or a salt thereof and/or glycosaminoglycan or a salt thereof for the manufacture of a preventing or treating agent for arthritis.
(24) Use of plant belonging to the genus Hydrangea or an extract of the plant and amino sugar or a salt thereof and/or glycosaminoglycan or a salt thereof for the manufacture of a food and drink or an additive for foods and drinks.
(25) Use of plant belonging to the genus Hydrangea or an extract of the plant and amino sugar or a salt thereof and/or glycosaminoglycan or a salt thereof for the manufacture of a food and drink or an additive for foods and drinks used for prevention or treatment of arthritis.
(26) Use of plant belonging to the genus Hydrangea or an extract of the plant and amino sugar or a salt thereof and/or glycosaminoglycan or a salt thereof for the manufacture of a feed or an additive for feeds.
(27) Use of plant belonging to the genus Hydrangea or an extract of the plant and amino sugar or a salt thereof and/or glycosaminoglycan or a salt thereof for the manufacture of a feed or an additive for feeds used for prevention or treatment of arthritis.

Examples of the plants belonging to the genus Hydrangea are Hydrangeau macrophylla Seringe, Hydrangea macrophylla Seringe var. otaksa Makino, Hydrangea macrophylla Seringe subsp. serrata Makino var. japonica Makino, Hydrangea macrophylla Seringe var. acuminata, Hydrangea macrophylla Seringe var. Thunbergii Makino, Hydrangea scandens Seringe, Hydrangea hirta Sieb. et Zucc., Hydrangea involucrata Sieb., Hydrangea sikokiana Maxim., Hydrangea paniculata Sieb., Hydrangea petiolaris Sieb. et Zucc., Hydrangea macrophylla Seringe subsp. serrata Makino var. amoena Makino, Hydrangea macrophylla Seringe subsp. serrata Makino var. angustata Makino, and the like. Among them, Hydrangea macrophylla Seringe var. Thunbergii Makino is preferably used.

With regard to the plant belonging to the genus Hydrangea, any of, for example, wild plant, plant obtained by cultivation, plant obtained by tissue culture, and the like may be used, so far as it is the plant belonging to the genus Hydrangea.

Examples of the plant are the parts such as leaves, flowers, branches, stems, fruits, roots, seeds, cultured cells, organs or callus and the like. It is also possible to use various treated matters prepared by physical, chemical or biological treatment of those parts.

The physical or chemical treatment includes drying such as sun-drying and air-drying as well as freeze-drying treatment, pulverizing treatment, extracting treatment. The physically or chemically treated matters include dried matters, freeze-dried matters, pulverized matters, extracted matters, and the like.

The biological treatment includes fermentation treatment, and the like. The biologically treated matters include fermented matters, and the like.

When Hydrangea macrophylla Seringe var. Thunbergii Makino is used as a plant belonging to the genus Hydrangea for example, Hydrangeae Dulcis Folium which is prepared by a drying treatment of Hydrangea macrophylla Seringe var. Thunbergii Makino after a fermenting treatment is preferably used.

Hereinafter, the plant belonging to the genus Hydrangea will be called the plant of the present invention.

Examples of the extract of the plant according to the present invention are substances which are obtained by methods for extracting the substances having a preventive or therapeutic effect for arthritis from the plant, such as extraction with various solvent and extraction with supercritical fluid from the plant.

With regard to the solvent used for extraction with solvent, any of, for example, aqueous medium and organic solvent may be used so far as it is a solvent which can extract a substance having a preventive or therapeutic effect for arthritis (hereinafter, may also be referred to as active ingredient). The solvent may be used alone or as a mixed solvent where two or more are combined.

With regard to an aqueous medium, water, aqueous solution of inorganic salt, buffer, and the like may be listed and water is preferred.

Examples of water are tap water, distilled water, deionized water, pure water, and the like.

Examples of the buffer are a phosphate buffer, a citrate buffer, and the like.

Examples of an inorganic salt for the aqueous solution of inorganic salt are sodium chloride, potassium chloride, calcium chloride, and the like.

With regard to the organic solvent, any solvent may be used so far as active ingredients can be extracted and its examples are monohydric aliphatic alcohol such as methanol, ethanol, 1-propanol, 2-propanol, 1-butanol and 2-butanol; dihydric aliphatic alcohol such as ethylene glycol and propylene glycol; trihydric aliphatic alcohol such as glycerol; alkyl acetate such as methyl acetate and ethyl acetate; aliphatic ketone such as acetone and ethyl methyl ketone; aliphatic ether such as dimethyl ether and diethyl ether; aliphatic hydrocarbon such as hexane, heptane and petroleum ether; alicyclic hydrocarbon such as cyclohexane; aromatic hydrocarbon such as toluene and benzene; halogenated aliphatic hydrocarbon such as chloroform, dichloromethane, 1,1,1,2-tetrafluoroethane and 1,1,2-trichloroethene; edible fat and oil such as sesame oil, corn oil, olive oil and cotton seed oil; liquefied aliphatic hydrocarbon such as methane, ethane, propane, propylene, butane and butylene; and dimethyl sulfoxide.

With regard to a sole solvent, aqueous medium, alcohol or aliphatic ketone is preferred. With regard to the alcohol, monohydric aliphatic alcohol is more preferred and ethanol is particularly preferred. With regard to an aliphatic ketone, acetone is preferred.

With regard to a mixed solvent, for example, an aqueous alcohol is preferred. An aqueous monohydric aliphatic alcohol is more preferred and an aqueous ethanol is particularly preferred. As to the water content, it is preferred to be not more than 70% (v/v) and, more preferably, not more than 40% (v/v).

With regard to the solvent, liquefied carbon dioxide and supercritical fluid carbon dioxide may be used.

In extracting with a solvent, apparatus which are commonly used for extraction with solvent such as stirrer, ultrasonic wave generator, reflux extractor, Soxhlet extractor, homogenizer and shaker may be used.

There is no particular limitation for the amount of the solvent used for the extraction with solvent and, for example, 0.1 to 10,000 part(s) by weight or, preferably, 1 to 100 part(s) by weight of the solvent is used to 1 part by weight of the plant of the present invention. With regard to the extracting temperature, although there is no particular limitation provided that it is the temperature of not lower than melting point and not higher than boiling point of the solvent, it is preferably 0 to 100°C and, more preferably, 20 to 90°C in the case of an aqueous medium while, in the case of organic solvent, it is preferably 0 to 1,000°C and, more preferably, 20 to 90°C. Although there is no particular limitation for the extracting time, it is preferably 1 minute to 1 week and, more preferably, 30 minutes to 1 day.

After completion of the extraction with a solvent, solid-liquid separation methods such as sedimentation, cake filtration, clear filtration, centrifugal filtration, centrifugal sedimentation, compression separation and filter press or, preferably, filtration is conducted to prepare an extracted liquid and that may be used as an extract. A residue prepared by the above solid-liquid separation methods may be further extracted with an extracting solvent to use as an extract.

When a supercritical fluid extraction (which may be merely called supercritical extraction) is conducted, an extracting fluid may be used solely, but it is also possible to use a mixture of an extracting fluid with an entrainer.

With regard to the extracting fluid, any of supercritical fluids such as carbon dioxide, ammonia, methanol, ethanol, isopropyl alcohol, ethane, propane, butane, pentane, hexane, dimethylbutane, benzene, dichlorodifluoromethane, dichlorofluoromethane, trichlorofluoromethane, dichlorotetrafluoromethane, chlorotrifluoromethane, diethyl ether, ethyl methyl ether, hexane and dinitrogen monoxide may be used and supercritical carbon dioxide is preferably used.

Incidentally, in the present invention, supercritical fluid (which may also be called supercritical gas) means fluid which is in a state beyond critical temperature and critical pressure.

With regard to an entrainer, any of monohydric aliphatic alcohols such as methanol and ethanol, water, aqueous monohydric aliphatic alcohol, acetone, hexane, and the like may be used and ethanol is preferably used. The amount of the entrainer to be added is preferably 0.1 to 50% (w/w) and, more preferably, 1 to 20% (w/w).

Although there is no particular limitation for the amount of extracting fluid to be used for the supercritical fluid extraction, it is used, for example, 5 to 1,000 parts by weight or, preferably, 20 to 50 parts by weight of extracting fluid as an accumulated amount to 1 part by weight of the plant of the present invention. If necessary, the extracting fluid may be circulated.

Although there is no particular limitation for the amount of the entrainer to be used for the supercritical fluid extraction, it is, for example, 0.001 to 100 part(s) by weight or, preferably, 1 to 20 part(s) by weight as an accumulated amount to 1 part by weight of the supercritical fluid.

A supercritical fluid extracting apparatus is used for the supercritical fluid extraction.

Temperature and pressure of the extracting tank used for the supercritical fluid extraction should be higher than the critical temperature and critical pressure, respectively, although there is no particular limitation for their upper limits.

Critical temperature and critical pressure vary depending upon the type of the compound and, in the case of carbon dioxide, temperature of the extracting tank is preferably 31.06 to 1,000°C and, more preferably, 31.1 to 60°C while pressure of the extracting tank is preferably 72.9 to 5,000 atm and, more preferably, 75 to 1,000 atm. Although there is no particular limitation for the pressure of the separating tank, it is preferably 1 to 5,000 atm and, more preferably, 1 to 1,000 atm.

Although there is no particular limitation for the temperature of the separating tank used for the supercritical fluid extraction, it is preferably -273.16 to 1,000°C and, more preferably, -80 to 200°C. Although there is no particular limitation for the extracting time, it is preferably 1 minute to 100 hours and, more preferably, 20 minutes to 5 hours.

After completion of the supercritical fluid extraction, an extracted liquid may be prepared by changing temperature and pressure of the separating tank, fillers, and the like. Examples of the separating method for the extract are isothermal vacuum method, isothermal gas-liquid separating method, isobaric heating method, isobaric cooling method, adsorbing method, and the like. After completion of the separating operation, the extract may be obtained as a solid or a liquid in the separating tank or, when an entrainer is used, it may be obtained as an entrainer solution.

The extract of plant of the present invention may also be such a thing that an extract obtained by an extracting method such as solvent extraction or supercritical fluid extraction from the plant is further concentrated or treated with a drying method, a purifying method, etc.

Examples of concentration or drying methods are heating concentration, freezing concentration, reverse osmosis concentration, vacuum concentration, freeze-drying, natural drying, hot-air drying, air-drying, air-blowing drying, spray drying, reduced-pressure drying, sun drying, vacuum drying, spray drying, fluidized-bed drying, foamed-bed drying, film drying using a drum drier, ultrasonic wave drying, electromagnetic wave drying, and the like, and vacuum concentration, spray drying method and freeze-drying method are preferably used.

In the preparation of the extract of the plant of the present invention, it may be possible to conduct a device for not to inactive the active ingredient such as addition of antioxidant, preservative and the like or adjustment of heating temperature.

The extract of the plant of the present invention may be prepared in such a manner that an extract residue prepared by extraction with various solvents, supercritical fluid extraction, and the like under a condition where the active ingredient is not or is hardly extracted from the plant of the present invention is further extracted by extraction with various solvents, supercritical fluid extraction, and the like under a condition where the active ingredient is able to be extracted. It is preferred to use a method where the residue obtained after extraction of the plant of the present invention or, preferably, dried product with an aqueous medium is extracted with alcohol, aqueous alcohol or aliphatic ketone.

Examples of the amino sugar or a salt thereof according to the present invention are glucosamine, galactosamine, neuraminic acid, N-acetylglucosamine, N-acetylgalactosamine, N-acetylneuraminic acid, N-glycolylneuraminic acid and a salt thereof in which glucosamine or a salt thereof is preferably used. Examples of the salt in a salt of the amino sugar are hydrochloride, sulfate and phosphate, etc.

With regard to glucosamine, a product prepared in such a manner, for example, that chitin obtained by removal of protein and removal of ash of shell of crustacean is hydrolyzed with concentrated hydrochloric acid, deacetylated, decolorized, filtered, concentrated, separated, washed and dried may be used or a commercially available one (such as Glucosamine KHF manufactured by Kyowa High-Foods) may be used.

Examples of the salt in glucosamine salt are hydrochloride, sulfate (such as glucosamine-6-sulfate), phosphate (such as glucosamine-6-phosphate), and the like.

Examples of the glycosaminoglycan in the present invention are hyaluronic acid, chondroitin, chondroitin sulfate, keratin sulfate, heparin, heparan sulfate, dermatan sulfate and a salt thereof in which chondroitin sulfate or a salt thereof is preferably used.

Examples of the salt in glycosaminoglycan salt are sodium salt, potassium salt, calcium salt, and the like.

Chondroitin sulfate is a kind of mucopolysaccharide generally distributed in connective tissues of an animal mainly in cartilage. In the tissues, it is bound to protein and is present as proteoglycan.

With regard to the chondroitin sulfate, it may be used as a purified one or in a form of proteoglycan, cartilage extract or dried cartilage powder.

In order to obtain chondroitin sulfate in a form of proteoglycan, cartilage of, for example, marine animals such as shark and whale, mammals such as cattle and pig, birds, or the like is used as a material, extraction is conducted according to a known method such as neutral salt method, alkali method, enzyme method or autoclave method (for example, a method mentioned in Japanese Published Unexamined Patent Application No. 2001-247602), fat, solids, and the like are removed and then dried. After fat and solids are removed, it is further treated for removal of protein using proteinase and purified according to a known method by means of alcohol precipitation which is described in Japanese Published Unexamined Patent Application No. 2001-247602, to thereby obtain chondroitin sulfate or a salt thereof in a pure state. A commercially available chondroitin sulfate or a salt thereof (such as Sodium Chondroitin Sulfate manufacture by Maruha) may be used.

Examples of a salt in chondroitin sulfate salt are sodium salt, potassium salt and calcium salt in which sodium salt is used preferably.

There is no particular limitation as to the arthritis to which the present invention can be applied, and examples include chlamydial arthritis, chronic absorptive arthritis, enteropathic arthritis, gonococcal arthritis, gouty arthritis, Jaccoud's arthritis, juvenile arthritis, Lyme arthritis, ochronotic arthritis, suppurative arthritis, osteoarthritis, shoulder periarthritis, arthritis caused by hyperkinesis, rheumatoid arthritis, and the like. The present invention is particularly effective for rheumatoid arthritis.

The preventing or treating agent of the present invention can be produced by any method which has been well known in the technical field of pharmaceutics in such a manner that a product prepared by the above-mentioned method comprising plant or an extract of the plant and amino sugar or a salt thereof and/or glycosaminoglycan or a salt thereof (hereinafter, referred to active ingredient of the present invention) is mixed, if necessary, with one or more pharmaceutically acceptable carrier(s).

With regard to the active ingredient of the present invention, any of a combination of plant of the present invention or an extract of the plant with amino sugar or a salt thereof, a combination of plant of the present invention or an extract of the plant with glycosaminoglycan or a salt thereof and a combination of plant of the present invention or an extract of the plant with amino sugar or a salt thereof and glycosaminoglycan or a salt thereof may be used and it is preferred to be a combination of plant of the present invention or an extract of the plant with amino sugar or a salt thereof and glycosaminoglycan or a salt thereof.

Further, the preventing or treating agent of the present invention may, if necessary, comprise ingredient for prevention or treatment of other diseases. Moreover, other ingredient effective for preventing or treating arthritis may be added thereto as well.

Examples of other ingredients effective for preventing or treating arthritis include boron, calcium, chromium, copper, magnesium, manganese, selenium, silicon, zinc, S-adenosylmethionine, collagen, collagen hydrolyzate, gelatin, gelatin hydrolyzate, bromelain, trypsin, chymotrypsin, papain, rutin, carotenoid, flavonoid, anti-oxidant vitamin, γ-linolenic acid, eicosapentaenoic acid, Boswellia, capsaicin, cat's claw, devil's claw, feverfew, ginger, nettles, niacinamide, turmeric, curcumin, and the like. Other ingredients effective for preventing or treating arthritis may be in a pure form or as a mixture containing them or as an extract.

It is desirable to administer a preventing or treating agent of the present invention through an effective route for preventing or treating arthritis and examples thereof are by oral administration and parenteral administration such as intravenous administration.

With regard to the dosage form, any of oral preparations such as tablets, powders, granules, pills, capsules, suspensions, emulsions, elixirs, syrups, liquid preparations, infusions, decoctions, extracts, tinctures and fluid extracts and parenteral preparations such as injections, drippings, creams and suppositories may be used although oral preparations are preferably used.

In preparing the oral preparations, additives such as excipient, binder, disintegrating agent, lubricant, dispersing agent, suspending agent, emulsifier, diluting agent, buffer, antioxidant and bacteria suppressing agent may be used.

When the dosage form of the oral preparation is tablets, powder, granules, and the like, the preparation may be prepared by addition of saccharides such as lactose, sugar, glucose, sucrose, mannitol and sorbitol; starch such as that of potato, wheat and corn; inorganic substances such as calcium carbonate, calcium sulfate, sodium hydrogen carbonate and sodium chloride; excipients such as crystalline cellulose and plant powder (e.g., licorice root powder and gentian powder); disintegrating agents such as starch, agar, gelatin powder, crystalline cellulose, carmellose sodium, carmellose calcium, calcium carbonate, sodium hydrogen carbonate and sodium alginate; lubricants such as magnesium stearate, talc, hydrogenated plant oil, Macrogol and silicone oil; binders such as polyvinyl alcohol, hydroxypropyl cellulose, methyl cellulose, ethyl cellulose, carmellose, gelatin and starch paste; surfactants such as fatty acid ester; plasticizers such as glycerol; and the like.

When dosage form of the oral preparation is a liquid preparation such as syrup, the preparation may be prepared by addition of diluents such as water, saccharides (e.g., sucrose, sorbitol and fructose), glycols (e.g., polyethylene glycol and propylene glycol) and oils (e.g., sesame oil, olive oil and soybean oil); antiseptics such as p-hydroxybenzoate; preservatives such as p-oxybenzoate derivatives (e.g., methyl p-oxybenzoate) and sodium benzoate; flavor such as strawberry flavor and peppermint; and the like.

The formulation suitable for oral administration, e.g. , extracts, tinctures or fluid extracts, may be prepared by using the extract obtained by extracting the plant body of the present invention with, for example, water, ethanol or a mixture of water and ethanol as such, or by concentrating the obtained extract.

The formulation suitable for parenteral administration preferably comprises a sterilized aqueous preparation containing an active ingredient of the present invention which is isotonic to the recipient's blood. For example, for injections, an injectable solution may be prepared using a carrier such as a salt solution, a glucose solution, or a mixture of a salt solution and a glucose solution.

In these parenteral preparations, it is also possible to add one or more of supplementary components for the oral preparations mentioned above, selected from diluents, antiseptics, preservatives, flavors, excipients, disintegrators, lubricants, binders, surfactants, and plasticizers.

The dose and frequency of the administration of the agent for preventing or treating arthritis of the present invention depend on the dosage form, age and body weight of the patient, and the symptom and degree of the disease to be treated, and the like. For example, in oral administration, it is administered preferably in an amount of 0.001 to 50 g and, more preferably, 0.005 to 10 g, once or several times a day for an adult as a dried amount of the plant of the present invention or an extract of the plant and amino sugar or a salt thereof and/or glycosaminoglycan or a salt thereof. In parenteral administration such as intravenous administration, it is administered preferably in an amount of 0.0005 to 50 g and, more preferably, 0.002 to 10 g, once or several times a day for an adult as a dried amount of the plant of the present invention or an extract of the plant and amino sugar or a salt thereof and/or glycosaminoglycan or a salt thereof. Period for the administration is usually one week to ten year and, preferably, one year to five years.

Meanwhile, the preventing or treating agent of the present invention may be used not only for humans but also for animals other than humans (hereinafter, may also be referred to as non-human animals). Dose and dosage frequency when used for non-human animals is preferably 0.002 to 10 g and, more preferably, 0.01 to 2 g as dried amount of the plant of the present invention or an extract of the plant and amino sugar or a salt thereof and/or glycosaminoglycan or a salt thereof per kg of body weight of the non-human animal once or several times a day. Period for the administration is usually one week to five years and, preferably, two weeks to two years.

Composition ratio by weight of the dried substance of the plant of the present invention or an extract of the plant to amino sugar or a salt thereof and/or glycosaminoglycan or a salt thereof in a preventing or treating agent of the present invention is from 1:100 to 100:1, preferably from 1:50 to 50:1 and, particularly preferably, from 10:1 to 1:10.

The preventing or treating agent of the present invention may be prepared in such a manner that the active ingredients of the present invention are contained in the same preparation, but it is also possible that separate preparations therefor are prepared and used as a preparation in a form of a kit.

When the active ingredients of the present invention are contained in the kit, it is not always necessary that the plant of the present invention or an extract of the plant is contained in each preparation constituting the kit and the preparations may be in any combination. An example of the combination of the preparations constituting the kit is a combination of a preparation containing the plant of the present invention or an extract of the plant with a preparation containing amino sugar or a salt thereof and/or glycosaminoglycan or a salt thereof although it is not limited thereto.

Each preparation contained in the kit may be present in any state so far as it is a state where each preparation is present separately. For example, each preparation may be separately packed or may be mixed in the same container.

When each preparation contained in the kit is separately administered, it is desirable that administration is conducted within a period where the active ingredient in the preparation has a high efficacy *in vivo.* For example, all preparations are administered within 8 hours and, preferably, within 2 hours for one administration.

The additives for foods and drinks of the present invention may be prepared in the same manner as the oral preparations of the present invention. Usually, other food additives may, if necessary, be added to or dissolved in the additives for foods and drinks of the present invention to prepare, for example, powder, granules, pellets, tablets and various kinds of liquid preparations.

The foods and drinks of the present invention may be processed and produced by a common process for foods and drinks except that the active ingredient of the present invention or the additive for foods and drinks of the present invention is added to foods and drinks.

The foods and drinks of the present invention may also be produced by granulation methods such as fluid bed granulation, stirring granulation, extrusion granulation, oscillating granulation, gas stream granulation, compression molding granulation, disruption granulation, spray granulation, and jet granulation; coating methods such as pan coating, fluid bed coating and dry coating; swelling methods such as puff drying, excess steam method, foam mat method and microwave heating method; extrusion methods such as using extruding granulator or extruder; and the like.

The foods and drinks of the present invention may be in any forms such as juice, soft drinks, tea, lactic acid bacterium drinks, milk products such as fermented milk, ice cream, butter, cheese, yogurt; processed milk and skim milk, meat products such as ham, sausage and hamburger, fish paste foods such as *kamaboko* (boiled fish paste), *chikuwa* (a kind of Japanese fish sausage) and *satsumaage* (deep-fried fish ball containing vegetable bits), egg products such as *dashimaki* (omelet with stock) and *tamago-dofu* (steamed beaten egg with soup stock), confectionary such as cookies, jelly, chewing gum, candy and snacks, bread, noodles, pickles, smoked fish and meat, dried fish, *tsukudani* (simmered meat in soy sauce and sugar), salted product, soup, seasonings, and the like.

The foods and drinks of the present invention may also be in a form such as powdered foods, sheet-shaped foods, bottled foods, canned foods, retort foods, capsule foods, tablet foods, liquid foods and drinkable preparations.

The foods and drinks of the present invention may also be used as health food or functional food having a preventing or treating effect for arthritis.

To the foods and drinks or the additives for foods and drinks of the present invention, it is possible, if required, to add one or more additive(s) generally used in foods and drinks, for example, sweetener, coloring agent, preservatives, thickening stabilizer, antioxidant, color-developing agent, bleaching agent, anti-fungal agent, gum base, bitter agents, enzymes, wax, sour agent, seasonings, emulsifier, nutrient supplements, additional materials for preparation, flavors, spice extracts, and the like.

Examples of the sweeteners are aspartame, licorice, stevia, xylose, Momordica grosvenori, and the like.

Examples of the coloring agent are carotenoid or turmeric pigment, flavonoid, caramel pigment, oriental gromurell pigment, spirulina pigment, chlorophyll, red sweet potato pigment, red Chinese yam pigment, perilla pigment, blueberry pigment, and the like.

Examples of the preservatives are sodium sulfite, benzoic acids, extract of Aralia cordata, extract of Styrax japonica, extract of Artemisia capillaris, sorbic acids, propionic acids, and the like.

Examples of the thickening stabilizers are gums such as gum arabic or xanthan gum, alginic acids, chitin, chitosan, aloe extract, guar gum, hydroxypropyl cellulose, casein sodium, corn starch, carboxymethyl celluloses, gelatin, agar, dextrin, methyl cellulose, polyvinyl alcohol, microfibrous cellulose, microcrystalline cellulose, seaweed cellulose, sodium polyacrylate, sodium polyphosphate, carrageenan, yeast cell wall, konjak extract, nata de coco, mannan, and the like.

Examples of the antioxidants are vitamin C, sodium ethylenediaminetetraacetate, calcium ethylenediaminetetraacetate, erythorbic acid, oryzanol, catechin, quercetin, clove extract, enzyme-treated rutin, apple extract, sesame oil extract, dibutylhydroxytoluene, fennel extract, horseradish extract, dropwort extract, tea extract, Tempeh extract, extract of Houttuynia cordata, tocotrienol, tocopherols, rapeseed oil extract, green coffee extract, sunflower seed, ferulic acid, butylhydroxyanisole, extract of blueberry leaves, propolis extract, hego-ginkgo extract, hesperetin, pepper extract, garden balsam extract, gallic acid, myrica extract, eucalyptus extract, rosemary extract, and the like.

An example of the color-developing agent is sodium sulfite, and the like.

An example of the bleaching agent is sodium sulfite, and the like.

An example of the anti-fungal agent is o-phenylphenol, and the like.

Examples of the gum bases are methyl acetylricinoleate, Japanese lacquer wax, ester gum, elemi resin, urucury wax, ozokerite, opopanax resin, kauri gum, carnauba wax, guaiacum resin, gutta katiau, gutta hangkang, gutta-percha, glycerin fatty acid ester, spermaceti, copaiba balsam, copal resin, gum, rice bran wax, sugar cane wax, shellac, jelutong, sucrose fatty acid ester, sorba, sorbitan fatty acid esters, talc, calcium carbonate, dammar resin, chicle, chilte, tunu, low molecular gum, paraffin wax, fir balsam, propylene glycol fatty acid ester, powdered pulp, powdered rice husks, jojoba wax, polyisobutylene, polybutene, microcrystalline wax, mastic, massaranduba chocolate, beeswax, calcium phosphate, and the like.

Examples of the bitter agents are isoalpha bitter acid, caffeine, kawaratake extract, cinchona extract, Amur cork extract, gentian extract, spice extract, enzyme-treated naringin, Jamaica quassia extract, theobromine, naringin, bitter ash extract, extract of Artemisia absinthium, isodonis extract, Himematsutake extract, borapet, methylthioadenosine, litchi extract, olive tea, sour orange extract, hop extract, mugwort extract, and the like.

Examples of the enzymes are amylase, trypsin, rennet, lactic acid bacteria, and the like.

Examples of the wax are Japanese lacquer wax, vegetable wax, and the like.

Examples of the sour agents are adipic acid, itaconic acid, citric acids and citrates, succinic acids and succinates, sodium acetate, tartaric acids and tartrates, carbon dioxide, lactic acid, phytic acid, fumaric acid, malic acid, phosphoric acid, and the like.

Examples of the seasonings are amino acids such as asparagine, aspartic acids, glutamic acid, glutamine, alanine, isoleucine, glycine, serine, cystine, tyrosine, leucine, and proline; nucleic acids such as sodium inosinate, sodium uridylate, sodium guanylate, sodium cytidylate, calcium ribonucleotide, and sodium ribonucleotide; organic acids such as citric acid and succinic acid; potassium chloride, sodium solution of low salt content prepared from salt lake water, crude potassium chloride from sea water, whey salt, tripotassium phosphate, dipotassium hydrogen phosphate, potassium dihydrogen phosphate, disodium hydrogen phosphate, sodium dihydrogen phosphate, trisodium phosphate, chlorella extract, and the like.

Examples of the emulsifying agents are fatty acid monoglyceride, sorbitan fatty acid ester, and the like.

Examples of the nutrient supplements are zinc salts, vitamin C, various amino acids, 5-adenylic acid, iron chloride, hesperidin, various kinds of burnt calcium, various kinds of unburnt calcium, dibenzoylthiamine, calcium hydroxide, calcium carbonate, thiamine hydrochloride, dunaliella carotene, tocopherol, nicotinic acid, carrot carotene, palm oil carotene, calcium pantothenate, vitamin A, hydroxyproline, calcium dihydrogen pyrophosphate, ferrous pyrophosphate, ferric pyrophosphate, ferritin, heme iron, menaquinone, folic acid, riboflavin, and the like.

Examples of the additional materials for preparation are processing aids such as acetone and ion exchange resin, extract of fig leaves, extract of rice straw ash, kaolin, glycerin fatty acid ester, mulberry extract, bone ash, perilla extract, ginger extract, various tannins, Phaffia extract, grape seed extract, ethanol, and the like.

Examples of the flavors are those as mentioned above.

Examples of the spice extracts are capsicum extract, garlic extract, and the like.

The amount of the active ingredient or the additives for foods and drinks of the present invention to be added to foods and drinks of the present invention is appropriately selected depending upon types of foods and drinks, effect expected by ingestion of the foods and drinks, and the like. Usually it is added in an amount of 0.01 to 100% by weight and, preferably, 0.1 to 100% by weight as a dried amount of the plant or an extract of the plant and amino sugar or a salt thereof and/or glycosaminoglycan or a salt thereof.

The ingestion of the foods and drinks of the present invention depends on the ingesting form, age and body weight of the ingesting person, and the like but it is orally administered or ingested preferably in an amount of 0.001 to 50 g and, more preferably, 0.005 to 10 g, once or several times a day per an adult as a dried amount of the plant of the present invention or an extract of the plant and amino sugar or a salt thereof and/or glycosaminoglycan or a salt thereof. The period for ingestion is usually from 1 day to 10 years and, preferably, from 2 weeks to 5 years.

The foods and drinks of the present invention may be prepared in such a manner that the active ingredients of the present invention are contained in the same foods and drinks, but it is also possible to produce as a set of foods and drinks.

When the active ingredients of the present invention are contained in the set, it is not always necessary that the plant of the present invention or an extract of the plant is contained in each food and drink constituting the set, and each food and drink may be in any combination. An example of the combination of each food and drink constituting the set is a combination of the foods and drinks containing the plant of the present invention or an extract of the plant with the food and drink containing amino sugar or a salt thereof and/or glycosaminoglycan or a salt thereof although it is not limited thereto.

Each food and drink contained in the set may be present in any state so far as it is a state where each is present separately. For example, each food and drink may be separately packed or may be mixed in the same container.

When each food and drink contained in the set is separately ingested, it is desirable that administration is conducted within a period where the active ingredient in each food and drink has a high efficacy in vivo. For example, all food and drink are ingested within 8 hours and, preferably, within 2 hours per ingestion.

The additives for feeds of the present invention may be prepared in the same manner as the oral preparations of the present invention. Usually, other additives for feed may, if necessary, be added to or dissolved in the additives for feed of the present invention to prepare, for example, powder, granules, pellets, tablets and various kinds of liquid preparations.

The feeds of the present invention may be a feed for animals belonging to any of mammals, birds, reptiles, amphibians and fishes so far as it is a feed containing the active ingredient of the present invention. For example, it is a feed for mammals such as pets such as dog, cat and mouse, livestocks such as cattle and pig, for poultry such as hen and turkey or for cultivated fishes such as sea bream and young yellowtail. Preferably, it is used as a feed for mammals.

The feeds of the present invention may be produced by a common process for feeds except that the active ingredient of the present invention or an additive for feed of the present invention is added to feed.

Materials for the feeds include cereals, chaff and bran, vegetable oil cakes, animal feed materials, other feed materials, purified products, a mixture thereof, and the like.

Examples of the cereals are milo, wheat, barley, oats, rye, brown rice, buckwheat, foxtail millet, broomcorn mellet, Japanese millet, corn, soybean, and the like.

Examples of the chaff and bran are rice bran, defatted rice bran, wheat bran, wheat middlings, wheat, wheat germ, barely bran, screening, pellets, corn bran, corn germ, and the like.

Examples of the vegetable oil cakes are soybean oil cake, soybean flour, linseed oil cake, cotton seed oil cake, peanut oil cake, safflower oil cake, coconut oil cake, palm oil cake, sesame oil cake, sunflower oil cake, rapeseed oil cake, kapok oil cake, mustard seed oil cake, and the like.

Examples of the animal feed materials are fish meal (such as northern ocean meal, imported meal, whole meal and coastal meal), fish soluble, meat meal, meat and bone meal, blood powder, degraded hair, bone meal, processed by-product for livestocks, feather meal, silk-worm pupa, skim milk powder, casein, dry whey, and the like.

Examples of the other feed materials are stems and leaves of plants (such as alfalfa, hay cube, alfalfa leaf meal and the powder of false acacia), processed industrial by-products of corn (such as corn gluten meal, corn gluten feed and corn steep liquor), processed starch product (such as starch), sugar, fermentation industrial products (such as yeast, beer cake, malt root, alcohol cake and soy source cake), agricultural by-products (such as processed citrus fruit cake, soybean curd cake, coffee cake and cocoa cake), cassava, broad bean, guar meal, seaweed, krill, spirulina, chlorella, minerals, and the like.

Examples of the purified products are proteins (such as casein and albumin), amino acids, sugars (such as starch, cellulose, sucrose and glucose), minerals, vitamins, and the like.

The feeds of the present invention may also be produced by granulation methods such as fluid bed granulation, stirring granulation, extrusion granulation, oscillating granulation, gas stream granulation, compression molding granulation, disruption granulation, spray granulation, and jet granulation; coating methods such as pan coating, fluid bed coating and dry coating; swelling methods such as puff drying, excess steam method, foam mat method and microwave heating method; and extrusion methods such as using extruding granulator or extruder; and the like.

The amount of the active ingredient or the additives for feed of the present invention to be added to the feeds of the present invention is appropriately selected depending upon types of feed, effect expected by ingestion of the feed, and the like. Usually it is added in an amount of 0.01 to 100% by weight and, preferably, 0.1 to 100% by weight as a dried amount of the plant or an extract of the plant and amino sugar or a salt thereof and/or glycosaminoglycan or a salt thereof.

The ingestion of the feeds of the present invention depend on the ingesting form, type of the ingesting animal, age and body weight of the ingesting animal, and the like and it is orally administered or ingested preferably in an amount of 0.002 to 10 g and, more preferably, 0.01 to 2g, once or several times a day per kg of body weight of the animal as a dried amount of the plant of the present invention or an extract of the plant and amino sugar or a salt thereof and/or glycosaminoglycan or a salt thereof. The period for ingestion is usually from 1 week to 5 years and, preferably, from 2 weeks to 2 years.

The additives for feed of the present invention may also be administered as an oral preparation for animals having a preventing and treating effect for arthritis in the same dose and administering period as the ingesting amount and ingesting period, respectively, for the feed as mentioned above.

The feeds for animals of the present invention may be prepared in such a manner that the active ingredients of the present invention are contained in the same feeds for animals, but it is also possible to produce as a set of feeds.

When the active ingredients of the present invention are contained in the set, it is not always necessary that the plant of the present invention or an extract of the plant is contained in each feed constituting the set, and each feed may be in any combination. An example of the combination of each feed constituting the set is a combination of the feed containing the plant of the present invention or an extract of the plant with the feed containing amino sugar or a salt thereof and/or glycosaminoglycan or a salt thereof although it is not limited thereto.

Each feed for animals contained in the set may be present in any state so far as it is a state where each is present separately. For example, each feed for animals may be separately packed or may be mixed in the same container.

When each feed for animals contained in the set is separately ingested, it is desirable that administration is conducted within a period where the active ingredient in each feed set has a high efficacy in vivo. For example, all feeds are ingested within 8 hours and, preferably, within 2 hours per ingestion.

When the active ingredients of the present invention are administered to human being or non-human animals according to the above-mentioned methods, arthritis of the human being or non-human animals may be prevented or treated. Best Mode for Carrying Out the Invention

### Example 1

To 1 kg of dried powder of amacha (Hydrangea Dulcis Folium) (manufactured by Shihira Shoten) was added 10 L of distilled water and extraction was conducted by stirring the mixture at room temperature for 1 hour. After the mixture was separated into filtrate and residue by filtration, 10 L of distilled water was added to the extract residue and extraction was conducted once again by stirring at room temperature for 1 hour. Filtrate was prepared by filtration and combined with the previously-prepared filtrate. The mixed filtrate was concentrated by an evaporator and then freeze-dried to give 200 g of a freeze-dried powder.

### Example 2

To 1 kg of dried powder of amacha was added 20 L of distilled water and extraction was started by stirring at 40°C. The extraction was finished when absorbance at 313 nm of the liquid prepared by diluting the extract with distilled water to an extent of 1/200 reached 0.15. Filtrate was removed by filtration to give an extract residue. Extraction was started by adding 20 L of 60% (v/v) aqueous solution of ethanol to the residue followed by stirring at 40°C. The extraction was finished when absorbance at 313 nm of the liquid prepared by diluting the extract with distilled water to an extent of 1/200 reached 0.22. The filtrate obtained by filtration was concentrated by an evaporator followed by subjecting to freeze-drying to give 70 g of freeze-dried powder.

### Example 3

Suppressive effect to arthritis was tested using model mice of arthritis induced by collagen [Nature, 1980, vol. 283, pages 666-668].

As the first administration of type-2 collagen, an equal mixture of type-2 collagen (manufactured by Collagen Technical Training Institute) and Freund's complete adjuvant (manufactured by Iatron, Inc.) emulsified with a homogenizer was administered to the root of the tail intracutaneously to a male DBA/1J mouse of 6 weeks age (bred at Charles Liver) at a dose of 100 µl/mouse. At day 21 after the first administration of type-2 collagen, as the 2nd administration of type-2 collagen, an equal mixture of type-2 collagen and Freund's complete adjuvant emulsified with a homogenizer was similarly administered to the root of the tail intracutaneously at a dose of 100 µl/mouse.

From the first day of administration of the type-2 collagen, commercially available powder feed CE-2 (manufactured by Clea Japan) was ingested to mice of group 1, CE-2 to which 0.5% by weight of amacha extract prepared in Example 2 was added was ingested to mice of group 2, CE-2 to which 1.0% by weight of glucosamine (D-glucosamine sulfate di(sodium chloride); manufactured by Miyako Kagaku) and 1.0% by weight of chondroitin sulfate (Sodium Chondroitin Sulfate; manufactured by Maruha) were added was ingested to mice of group 3 and CE-2 to which 0.25% by weight of amacha extract prepared in Example 2, 0.5% by weight of glucosamine and 0.5% by weight of chondroitin sulfate were added was ingested to mice of group 4. CE-2 was ingested to an untreated group to which no type-2 collagen was administered.

On the 27th, 30th and 34th days after the first administration of type-2 collagen, degree of onset of arthritis was scored according to the following index. Thus, scoring was carried out by applying 0-4 points with respect to one paw of the mouse in such a manner that point 0 for no symptom, point 1 for light swelling on one finger or on ankle, point 2 for swelling on 1 to 3 finger(s) or on ankle, point 3 for swelling on 3 to 5 fingers and on ankle and point 4 for swelling on all fingers and on ankles. Then the total score of 4 paws of the mouse, that is, point 0 to 16, was recorded. The data are shown in terms of mean value ± standard error (n = 20).

Result of the scoring for arthritis is shown in Table 1.

**Table 1**

| Additive(s) | | Scores for Arthritis After | | | |
|---|---|---|---|---|---|
| | | 0 day | 27 days | 30 days | 34 days |
| Untreated Gr | | 0.00 ± 0.00 | 0.00 ± 0.00 | 0.00 ± 0.00 | 0.00 ± 0.00 |
| Group 1 | | 0.00 ± 0.00 | 6.65 ± 0.48 | 11.70 ± 0.56 | 13.30 ± 0.59 |
| Group 2 | Amacha extract (0.5%) | 0.00 ± 0.00 | 5.44 ± 0.51 | 10.11 ± 0.75 | 11.56 ± 0.72 |
| Group 3 | Glucosamine (1.0%) and Chondroitin Sulfate (1.0%) | 0.00 ± 0.00 | 5.60 ± 0.41 | 10.65 ± 0.64 | 12.30 ± 0.60 |
| Group 4 | Amacha extract (0.25%), Glucosamine (0.5%) and Chondroitin Sulfate (0.5%) | 0.00 ± 0.00 | 4.00 ± 0.68 | 8.29 ± 1.11 | 10.56 ± 1.04 |

As shown in Table 1, Groups 2 and 3 showed a weak suppression for an increase in the arthritis scores with lapse of time as compared with group 1. Now, group 4 in which amacha extract in one-half amount of Group 2 and glucosamine and chondroitin sulfate each in each one-half amount of Group 3 were combined and ingested significantly suppressed an increase in the arthritis scores with lapse of time as compared with Groups 2 and 3.

The result shows that the effect in Group 4 is far higher than the effect presumed from the result of Groups 2 and 3 and that, when glucosamine and chondroitin sulfate are combined with amacha extract, arthritis can be synergistically suppressed.

### Example 4

The following compositions were mixed to prepare a preventing or treating agent for arthritis.

| | |
|---|---|
| Freeze-dried powder prepared in example 2 | 9.8 g |
| Glucosamine (manufactured by Miyako Kagaku) | 19.6 g |
| Chondroitin sulfate (manufactured by Maruha) | 19.6 g |
| Pinedex #3 (manufactured by Matsutani Kagaku Kogyo) | 49 g |
| Ferric pyrophosphate (Tetsugen; manufactured by Kokusan Kagaku) | 0.1 g |
| Phoscal EFC (Calcium source; manufactured by Nikko Fine Products) | 1 g |
| Vitamin Mix (manufactured by Merck) | 1 g |

### Example 5

A preventing or treating agent (20 g) prepared in Example 4 was dispersed in 180 ml of water to give a preventing or treating drink for arthritis.

### Example 6

Thirty cookies were prepared according to the following ingredients.

| | |
|---|---|
| Soft wheat flour | 100 g |
| Starch | 74 g |
| Water | 14 g |
| Freeze-dried powder prepared in Example 2 | 6 g |
| Glucosamine (manufactured by Miyako Kagaku) | 12 g |
| Chondroitin sulfate (manufactured by Maruha) | 12 g |
| Baking powder | 2 teaspoonful |
| Salt | 2 teaspoonful |
| Egg | one |
| Butter | 80 g |
| Milk | 2 tablespoonful |

### Example 7

The following feed was prepared.

| | |
|---|---|
| Sucrose (manufactured by Kishida Kagaku) | 20.0 wt% |
| Corn oil (manufactured by Nakarai Tesque) | 5.0 wt% |
| Choline bitartrate (manufactured by Tokyo Kasei) | 0.4 wt% |
| Corn starch (manufactured by Nichiden Kagaku) | 39.1 wt% |
| AIN-76 vitamin (manufactured by Oriental Yeast) | 1.0 wt% |
| AIN-76 mineral (manufactured by Oriental Yeast) | 3.5 wt% |
| Cellulose (manufactured by Oriental Yeast) | 5.0 wt% |
| Casein (manufactured by Wako Pure Chemical) | 25.0 wt% |
| Freeze-dried powder prepared in Example 1 | 0.2 wt% |
| Glucosamine (manufactured by Miyako Kagaku) | 0.4 wt% |
| Chondroitin sulfate (manufactured by Maruha) | 0.4 wt% |

### Industrial Applicability

In accordance with the present invention, it is now possible to provide a preventing or treating agent for arthritis or foods and drinks, additives for foods and drinks, feeds or additives for feeds for prevention or treatment of arthritis comprising plant belonging to the genus Hydrangea or an extract of the plant and amino sugar or a salt thereof and/or glycosaminoglycan or a salt thereof as active ingredients.

## Claims

1. A preventing or treating agent for arthritis which comprises plant belonging to the genus Hydrangea or an extract of the plant and amino sugar or a salt thereof and/or glycosaminoglycan or a salt thereof as active ingredients.

2. The preventing or treating agent for arthritis according to claim 1, wherein the plant belonging to the genus Hydrangea is Hydrangea macrophylla Seringe var. Thunbergii Makino.

3. The preventing or treating agent for arthritis according to claim 2, wherein the plant of Hydrangea macrophylla Seringe var. Thunbergii Makino is Hydrangeae Dulcis Folium.

4. The preventing or treating agent for arthritis according to any of claims 1 to 3, wherein the amino sugar is glucosamine or a salt thereof.

5. The preventing or treating agent for arthritis according to any of claims 1 to 4, wherein the glycosaminoglycan is chondroitin sulfate or a salt thereof.

6. A food and drink or an additive for foods and drinks comprising plant belonging to the genus Hydrangea or an extract of the plant and amino sugar or a salt thereof and/or glycosaminoglycan or a salt thereof.

7. The food and drink or the additive for foods and drinks according to claim 6, wherein it is used for prevention or treatment of arthritis.

8. The food and drink or the additive for foods and drinks according to claim 6 to 7, wherein the plant belonging to the genus Hydrangea is Hydrangea macrophylla Seringe var. Thunbergii Makino.

9. The food and drink or the additive for foods and drinks according to claim 8, wherein the plant of Hydrangea macrophylla Seringe var. Thunbergii Makino is Hydrangeae Dulcis Folium.

10. The food and drink or the additive for foods and drinks according to any of claims 6 to 9, wherein the amino sugar is glucosamine or a salt thereof.

11. The food and drink or the additive for foods and drinks according to any of claims 6 to 10, wherein the glycosaminoglycan is chondroitin sulfate or a salt thereof.

12. A feed or an additive for feeds comprising plant belonging to the genus Hydrangea or an extract of the plant and amino sugar or a salt thereof and/or glycosaminoglycan or a salt thereof.

13. The feed or the additive for feeds according to claim 12, which is used for prevention or treatment of arthritis.

14. The feed or the additive for feeds according to claim 12 or 13, wherein the plant belonging to the genus Hydrangea is Hydrangea macrophylla Seringe var. Thunbergii Makino.

15. The feed or the additive for feeds according to claim 14, wherein the plant of Hydrangea macrophylla Seringe var. Thunbergii Makino is Hydrangeae Dulcis Folium.

16. The feed or the additive for feeds according to any of claims 12 to 15, wherein the amino sugar is glucosamine or a salt thereof.

17. The feed or the additive for feeds according to any of claims 12 to 16, wherein the glycosaminoglycan is chondroitin sulfate or a salt thereof.

18. A preventing or treating method for arthritis, which comprises administering plant belonging to the genus Hydrangea or an extract of the plant and amino sugar or a salt thereof and/or glycosaminoglycan or a salt thereof.

19. The preventing or treating method for arthritis according to claim 18, wherein the plant belonging to the genus Hydrangea is Hydrangea macrophylla Seringe var. Thunbergii Makino.

20. The preventing or treating method for arthritis according to claim 19, wherein the plant of Hydrangea macrophylla Seringe var. Thunbergii Makino is Hydrangeae Dulcis Folium.

21. The preventing or treating method for arthritis according to any of claims 18 to 20, wherein the amino sugar is glucosamine or a salt thereof.

22. The preventing or treating method for arthritis according to any of claims 18 to 21, wherein the glycosaminoglycan is chondroitin sulfate or a salt thereof.

23. Use of plant belonging to the genus Hydrangea or an extract of the plant body and amino sugar or a salt thereof and/or glycosaminoglycan or a salt thereof for the manufacture of a preventing or treating agent for arthritis.

24. Use of plant belonging to the genus Hydrangea or an extract of the plant and amino sugar or a salt thereof and/or glycosaminoglycan or a salt thereof for the manufacture of a food and drink or an additive for foods and drinks.

25. Use of plant belonging to the genus Hydrangea or an extract of the plant and amino sugar or a salt thereof and/or glycosaminoglycan or a salt thereof for the manufacture of a food and drink or an additive for foods and drinks used for prevention or treatment of arthritis.

26. Use of plant belonging to the genus Hydrangea or an extract of the plant and amino sugar or a salt thereof and/or glycosaminoglycan or a salt thereof for the manufacture of a feed or an additive for feeds.

27. Use of plant belonging to the genus Hydrangea or an extract of the plant and amino sugar or a salt thereof and/or glycosaminoglycan or a salt thereof for the manufacture of a feed or an additive for feeds used for prevention or treatment of arthritis.
